(19) Europäisches Patentamt
European Patent Office
Office européen des brevets

(11) **EP 1 754 698 B1**

(12) **EUROPÄISCHE PATENTSCHRIFT**

(45) Veröffentlichungstag und Bekanntmachung des Hinweises auf die Patenterteilung:
**17.12.2008 Patentblatt 2008/51**

(51) Int Cl.:
**C07C 263/10** (2006.01)     **C07C 265/14** (2006.01)

(21) Anmeldenummer: **06015211.3**

(22) Anmeldetag: **21.07.2006**

(54) **Verfahren zur Gasphasenphosgenierung**

Process for gas phase phosgenation

Procédé d'une phosgénation en phase gaseuse

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.08.2005 DE 102005036870**

(43) Veröffentlichungstag der Anmeldung:
**21.02.2007 Patentblatt 2007/08**

(73) Patentinhaber: **Bayer MaterialScience AG
51368 Leverkusen (DE)**

(72) Erfinder:
• **Sanders, Josef, Dr.
51375 Leverkusen (DE)**
• **Brümmer, Hanno, Dr.
40237 Düsseldorf (DE)**
• **Laue, Jörg, Dr.
41541 Dormagen (DE)**
• **Sojka, Bernd, Dr.
50733 Köln (DE)**
• **Eichmann, Marcus, Dr.
40545 Düsseldorf (DE)**
• **Haverkamp, Verena, Dr.
51467 Bergisch Gladbach (DE)**

(56) Entgegenhaltungen:
EP-A2- 1 319 655          WO-A-00/56160
WO-A-01/65194            WO-A-20/05016512

• M. FICHTER ET AL: "MICROSTRUCTURE DEVICES FOR APPLICATIONS IN THERMAL AND CHEMICAL PROCESS ENGINEERING, HEAT AND TRANSPORT PHENOMENA IN MICROSYSTEMS" PROC. OF THE INTERNAT. CONF.,BANFF, OCT. 15-20, 2000, Seiten 41-53, XP009077378

## Beschreibung

Gegenstand der vorliegenden Erfindung ist ein Verfahren zur Phosgenierung von Aminen in der Gasphase, wobei zur Verdampfung der Amine eine spezielle Art von Wärmetauschern verwendet wird.

In der EP-A 0 289 840 wird ein Verfahren zur Herstellung von (cyclo)aliphatischen Diisocyanaten durch Phosgenierung der entsprechenden, dampfförmigen (cyclo)aliphatischen Diamine bei 200°C bis 600°C beschrieben. Phosgen wird in stöchiometrischem Überschuss zugeführt. Die überhitzten Ströme von dampfförmigem (cyclo)aliphatischem Diamin bzw. (cyclo)aliphatischem Diamin-Inertgas-Gemisch einerseits und von Phosgen andererseits werden kontinuierlich in einen zylindrischen Reaktionsraum geleitet, dort miteinander vermischt und zur Reaktion gebracht. Die exotherme Phosgenierreaktion wird unter Aufrechthaltung einer turbulenten Strömung durchgeführt.

In EP-A 928 785, EP-A 1 319 655, EP-A 1 555 258, EP-A 1 275 639, EP-A 1 275 640, EP-A 1 403 248 und EP-A 1 526 129 werden zu dieser Technologie spezielle Ausführungsformen beschrieben, welche sich allerdings auf den Reaktor an sich und die Reaktionsführung beziehen, ohne näher auf die Verdampfertechnologie, die zur Vorbehandlung der Edukte eingesetzt wird, einzugehen.

Üblicherweise werden zur Erhitzung und Verdampfung der eingesetzten Edukte Amine und Phosgen Röhrbündelwärmetauscher, Plattenwärmetauscher oder Fallfilmverdampfer vorzugsweise mit Umpumpkreislauf eingesetzt. Zur Überhitzung der gasförmigen Amine werden elektrisch oder mit Wärmeträgerölen betriebene Heizregister verwendet. Diese Apparate weisen jedoch den Nachteil auf, dass die hierbei auftretenden relativ großen Filmdicken den Stoff und Wärmetransport ungünstig beeinflussen, was eine größere Verweilzeit erfordert. Hierdurch kommt es insbesondere bei der Verdampfung und Überhitzung von aliphatischen Aminen zu einer partiellen Zersetzung unter Ammoniak-Abspaltung. Dies vermindert nicht nur die Ausbeute, sondern es bilden sich bei der nachfolgenden Phosgenierungsreaktion auch Ablagerungen von Ammoniumchlorid in Rohrleitungen und Reaktor. Die Anlagen müssen daher relativ häufig gereinigt werden, wobei entsprechende Produktionsverluste entstehen.

So genannte Mikrowärmetauscher oder Mikroverdampfer wurden bisher in WO 2005/016512 nur im Zusammenhang mit der destillativen Entfernung von Verbindungen aus Flüssigkeitsmischungen beschrieben. Auf dem technischen Gebiet der Gasphasenphosgenierung von Aminen zu Isocyanaten wurden diese Apparate jedoch in keiner Hinsicht beschrieben oder auf deren möglichen Vorzüge hingewiesen.

Aufgabe der vorliegenden Erfindung war es daher, ein Verfahren zur Phosgenierung von Aminen in der Gasphase bereitzustellen, bei dem die vorstehend genannten Nachteile konventioneller Wämetauscher bzw.

Verdampfer nicht auftreten.

Dies wurde nun gerade durch den Einsatz von Milli- wärmetauschern für die Flüssigerhitzung, Verdampfung und Gasüberhitzung der Amine erreicht.

Gegenstand der Erfindung ist daher ein Verfahren zur Herstellung von Isocyanaten durch Phosgenierung von Aminen in der Gasphase, bei dem zur Flüssigerhitzung, Verdampfung und/oder Gasüberhitzung der Amine ein oder mehrere Wärmetauscher mit einer volumenspezifischen Wärmetauscherfläche für die Aminseite von mindestens 1.000 $m^2/m^3$ eingesetzt werden, die zur Strömungsführung der Amine Kanäle aufweisen, die einen hydraulischen Durchmesser von 1000 bis 10.000 $\mu m$ besitzen.

Solche Wärmetauscher bzw. Verdampfer sind abhängig vom Durchmesser der Kanäle auch als Milliwärmetauscher oder -verdampfer (bei Durchmessern der Kanäle für die Strömungsführung von $\geq$ 1000 $\mu m$) bekannt.

Dieser erfindungswesentlichen Verdampfer bzw. Wärmetauscher besitzen bei gleicher Leistung gegenüber konventionellen Wärmetauschern ein geringeres Volumen, wodurch die Verweilzeit und damit auch die thermische Belastung der Amine erheblich reduziert wird. Typischerweise ist die Verdampfung und damit die Verweilzeit 10 bis 100 mal schneller bzw. kürzer als bei konventionellen Systemen.

Als Amine können grundsätzlich alle dem Fachmann bekannten Verbindungen mit primären Aminogruppen zur Phosgenierung eingesetzt werden. Bevorzugt sind dies jedoch solche Verbindungen mit wenigstens 2, bevorzugt 2 oder 3 $NH_2$-Gruppen, die aliphatisch, cycloaliphatisch oder aromatisch gebunden sein können.

Beispielsweise geeignete Amine sind die reinen Isomere oder die Isomerengemische des Diaminobenzols, des Diaminotoluols, des Diaminodimethylbenzols, des Diaminonaphthalins sowie des Diaminodiphenylmethans, bevorzugt sind 2,4/2,6-Toluylendiamin-Gemische der Isomerenverhältnisse 80/20 und 65/35 oder das reine 2,4-Toluylendiamin-Isomer.

Geeignete aliphatische bzw. cycloaliphatische Amine sind 1,4-Diaminobutan, 1,6-Diaminohexan (HDA), 1,11-Diaminoundecan, 1-Amino-3,5,5-trimethyl-5-aminomethylcyclohexan (IPDA), 4,4'-Diaminodicyclohexylmethan, 4,4'-Diaminodicyclohexylpropan-2,2 oder 1,8-Diamino-4-(aminomethyl)octan (Triaminononan).

Besonders bevorzugt sind jedoch Di- und/oder Triamine der vorstehend genannten Art mit ausschließlich aliphatisch oder cycloaliphatisch gebundenen Aminogruppen wie Isophorondiamin (IPDA), Hexamethylendiamin (HDA), Bis(p-aminocyclohexyl)methan, (PACM 20) oder 1,8-Diamino-4-(aminomethyl)octan (Triaminononan).

Das Grundprinzip der Gasphasenphosgenierung ist in den bereits genannten EP-Anmeldungen ausführlich beschrieben.

Dabei werden die zu phosgenierenden flüssigen Amine wie das Phosgen jeweils separat zunächst verdampft, gegebenenfalls mit einem Inertgas oder mit den Dämpfen eines inerten Lösungsmittels verdünnt, gegebenenfalls gasüberhitzt und dann kontinuierlich in einem typischerweise auf 200 bis 600°C erhitzten, meist zylindrischen Reaktionsraum ohne sich bewegende Teile unter Aufrechterhaltung einer turbulenten Strömung miteinander zur Reaktion gebracht. Das den Reaktionsraum kontinuierlich verlassende Gasgemisch wird dann bevorzugt mit Hilfe eines inerten, flüssigen Lösungsmittels, welches bei einer Temperatur oberhalb der Zersetzungstemperatur des dem Amin entsprechenden Carbamidsäurechlorids gehalten wird unter Gewinnung einer Lösung des entsprechenden Isocyanats in diesem Lösungsmittel abkühlt und das in dem inerten Lösungsmittel gelöst vorliegende Isocyanat beispielsweise destillativ abgetrennt.

Im Rahmen der Erfindung geeignete Milli- oder Mikrowärmetauscher sind beispielsweise Stapelkanalmikro- und Stapelkanalmilliwärmetauscher. Werden diese zur Verdampfung eingesetzt, spricht man entsprechend von Stapelkanalmikro- und Stapelkanalmilliverdampfern. Diese sind typischerweise schichtweise aus dünnen Metall-Platten aufgebaut, wobei jede Platte eine Vielzahl von parallelen Kanälen zur Strömungsführung aufweist. Die Platten sind beispielsweise kreuzweise zueinander angeordnet, so dass die Kanäle einer Platte zu den Kanälen der jeweils darunter und/oder darüber befindlichen Platte senkrecht stehen. Entsprechend werden in solchen Anordnungen das Wärmeübertragungsmittel und das Stoffgemisch im Kreuzstromprinzip durch den Wärmetauscher bzw. Verdampfer geleitet: Jede zweite Schicht ist von dem Heizmedium bzw. von dem Stoffgemisch durchströmt.

Die Platten besitzen z.B. eine Dicke von 100 bis 1.000 $\mu$m. Die Einzelkanäle haben typischerweise eine Länge von jeweils 0,5 bis 400 cm, vorzugsweise von 1 bis 150 cm.

Derartige Stapelkanalwärmetauscher sind als Milliwärmetauscher für das erfindungsgemäße Verfahren geeignet.

Unabhängig von der Geometrie der Kanäle der Milliwärmetauscher (bzw. verdampfer) wird im Sinne der vorliegenden Erfindung der hydraulische Durchmesser (D) als kennzeichnende Größe verwendet. Der hydraulische Durchmesser ist gegeben durch die vierfache Querschnittsfläche (F) dividiert durch den Umfang (U) des Querschnitts:

$$D = 4\ F/U$$

Solche Stapelkanalwärmetauscher werden beispielsweise vom Forschungszentrum Karlsruhe angeboten und werden unter anderem in K. Schubert, J. Brandner, M. Fichtner, G. Linder, U. Schygulla, A. Wenka, Microstructure devices for applications in thermal and chemical process engineering, Heat and Transport Phenomena in Microsystems: Proc. Of the Internat. Conf., Banff, Oct. 15-20, 2000 beschrieben.

Statt der oben beschriebenen Stapelkanalwärmetauscher bzw. -verdampfer können im erfindungsgemäßen Verfahren auch spezielle Rohrwärmetauscher bzw. -verdampfer eingesetzt werden, die die vorstehend definierten Kriterien der volumenspezifischen Wärmetauscherfläche und des hydraulischen Durchmessers der Kanäle zur Strömungsführung der Amine erfüllen. Daher werden sie als Kanalrohrwärmetauscher bezeichnet.

Diese weisen anstatt von Stapelkanälen ein oder mehrere parallel in einem Mantelraum angeordnete Rohre zur Strömungsführung der Amine auf. Der Außenraum wird dabei vom Wärmeträger durchströmt. Solche speziellen den vorstehenden Kriterien entsprechende Rohrwärmetauscher können ein einzelnes oder mehrere parallel zueinander angeordnete Kanalrohre aufweisen. Der Außenraum solcher Rohrwärmetauscher wird bevorzugt mit Umlenkblechen bestückt, welche die Strömungsbedingungen und damit den Wärmeübergang verbessern. Das Wärmeträgermedium kann dabei den Außenraum sowohl im Gleich- als auch im Gegenstrom durchströmen.

Die in solchen speziellen Rohrwärmetauschern eingesetzten Kanalrohre haben üblicherweise eine Länge von jeweils 10 cm bis 400 cm, vorzugsweise von 30 bis 150 cm. Die Wandstärke der Rohre liegt üblicherweise bei 0,5 bis 6 mm.

Derartige Rohrwärmetauschern, die die erfindungswesentlichen Kriterien der volumenspezifischen Wärmetauscherfläche und des hydraulischen Durchmessers der Kanäle zur Strömungsführung der Amine erfüllen sind grundsätzlich sowohl als Milli- als auch als Mikrowärmetauscher für das erfindungsgemäße Verfahren geeignet. Bevorzugte Rohrwärmetauscher sind jedoch Millikanalrohrwärmetauscher.

Werden Milliwärmetauscher bzw. -verdampfer der vorstehend beschriebenen Art, beispielsweise in Form von Stapelkanalmilliwärmetauschern bzw. Millikanalrohrwärmetauscher, eingesetzt, beträgt der hydraulische Durchmesser der Kanäle zur Führung des Aminstromes 1.000 bis 10.000 $\mu$m, bevorzugt 2.000 bis 5.000 $\mu$m.

Gleichzeitig beträgt die auf das Volumen der Aminkanäle bezogene Wärmeaustauschfläche in den Milliwärmetauschern bevorzugt $1 * 10^3$ bis $1 * 10^5\ m^2/m^3$ besonders bevorzugt 1 bis $2 * 10^3\ m^2/m^3$.

In Stapelkanalmilliwärmetauschern weisen auch die Kanäle für die Führung des Heizmediums bevorzugt einen hydraulischen Durchmesser von 5 bis 10.000 $\mu$m, bevorzugt von 5 bis 1000 $\mu$m, besonders bevorzugt von 30 bis 500 $\mu$m auf.

Die Kanäle der Milliwärmetauscher für die Durchleitung der Amine und des Heizmediums können beliebige geometrische Formen aufweisen. Der Querschnitt der Kanäle kann z.B. rund, halbrund, eckig, rechteckig oder dreieckig sein. Bevorzugt sind die Kanäle rechteckig oder dreieckig und bei Millikanalrohrwärmetauschern auch oval.

Grundsätzlich können die Kanäle zur Strömungsführung auch Einbauten enthalten. Dadurch wird der Wärmeübergang gegenüber solchen Systemen erhöht, die keine solche Einbauten enthalten. Die Einbauten können aber auch fest mit den Kanälen verbunden sein. In diesem Fall wirken die Einbauten zusätzlich als Wärmeübertragungsrippen, wodurch die Wärmeübertragung zusätzlich begünstigt wird.

Beispiel solcher Einbauten können Schichtenstrukturen sein. Solche Strukturen bestehen aus mindestens drei Schichten, wobei jede strukturierte Schicht im eingebauten Zustand eine Vielzahl von Öffnungen aufweist, die in mindestens einer Längsreihe angeordnet sind und sich die Öffnungen einer mittleren Schicht mit mindestens drei Öffnungen einer jeweils benachbarten Schicht überschneiden, und dass die Abfolge der sich überschneidenden Öffnungen einen Strömungskanal in Längsrichtung oder Querrichtung der Schichten bildet. Solche Strukturen können durch den Einsatz von Blechen mit einer Abfolge schräg angeordneter Öffnungen gebildet werden, wie sie in EP-A 1 284 159 beschrieben werden. Anstelle von Blechen mit Öffnungen können auch Kammprofilschichten, wie sie in EP-A1 486 749 beschrieben werden, eingesetzt werden. Hierbei bietet sich vor allem der Einsatz von symmetrischen, zweiseitigen Kammprofilen an, welche den Kanalinnenraum in zwei getrennte parallele Kanalzonen unterteilen. Die Öffnungen der Blechstrukturen bzw. die Kammzähne der Kammstrukturen werden in einem Winkel von 5 bis 85°, bevorzugt 30 bis 60° zur Hauptströmungsrichtung angeordnet. Bevorzugt beträgt die Zahl der Öffnungen bzw. Kammzähne in einer strukturierten Schicht zur Bildung einer Reihe von Öffnungen mindestens 50, besonders bevorzugt mindestens 200, ganz besonders bevorzugt mindestens 500.

Ein aus strukturierten Schichten gefüllter Milliwärmetauscherkanal ist im Hinblick auf die Rückvermischung und das Temperaturprofil insbesondere dann vorteilhaft, wenn die Kanallänge (L) mit dem hydraulischen Durchmesser des Kanals (D) ein L/D-Verhältnis von größer 10, bevorzugt größer 100 und besonders bevorzugt größer 500 bildet.

Zum Einsatz von Schichtstrukturen sind besonders Millikanäle mit rechteckigem oder ovalem Querschnitt geeignet.

Bevorzugt werden solche Einbauten in Milliverdampfern- bzw. -wärmetauschern eingesetzt, also solchen Vorrichtungen zur Aufheizung, Verdampfung und/ oder Überhitzung, welche Kanäle zur Strömungsführung der Amine von ≥1000 μm aufweisen.

Die Schichtstruktureinbauten für solche Milliwärmetauscher besitzen typischerweise eine Dicke von 0,1 bis 3 mm, vorzugsweise von 0,5 bis 1,5 mm. Die Kanäle, in die die Strukturen eingebaut werden, haben typischerweise eine innere Höhe von 1 bis 10 mm, vorzugsweise von 2 bis 5 mm, und eine Breite von 5 bis 50 mm, vorzugsweise von 10 bis 30 mm.

In Stapelkanalmilliwärmetauschern können nicht nur die Kanäle zur Strömungsführung der Amine derart ausgestaltet sein, sondern auch diejenigen Kanäle, durch welche das Heizmedium geführt wird. Dies kann sinnvoll sein, um den Wärmeübergang auch auf der Wärmeträgerseite zu verbessern.

Der Milliwärmetauscher bzw. Milliverdampfer kann aus einem beliebigen metallischen Werkstoff gefertigt sein, z.B. Stahl, Edelstahl, Titan, Hastelloy, Inconel oder anderen metallischen Legierungen.

Als Heizmedium können die üblichen Heizmedien wie Wasserdampf, Druckwasser oder Wärmeträgeröle eingesetzt werden.

Die Temperatur, bei der die erfindungsgemäß einzusetzenden Erhitzer-Wärmetauscher bzw. Verdampfer-Wärmetauscher betrieben werden, richtet sich nach dem Siedepunkt des zu verdampfenden Amins. Ziel ist es, nach Durchströmen des Erhitzer-Wärmetauschers eine Temperatur knapp unterhalb der Siedetemperatur des Amins und nach Durchtritt durch den Verdampfer einen vollständigen Übergang des zuvor flüssigen Amins in die Gasphase zu erreichen und gegebenenfalls noch im selben oder in einem weiteren Wärmetauscher das gasförmige Amin zu überhitzen. Dabei wird bewusst auf Kreislaufströmungen durch die Apparate verzichtet, so dass die Apparate nur einmalig von Amin durchströmt werden. Dies hat den Vorteil, dass auch auf das Volumen von sonst notwendigen Pumpenvorlagen verzichtet werden kann und die Verweilzeit im Bereich hoher Temperaturen weiter verringert wird. Die exakten Druck- und Temperaturbedingungen sind durch den Fachmann leicht anhand von Routineexperimenten zu ermitteln.

Bei der Verdampfung des Phosgens vor Eintritt in den Reaktor wird bevorzugt eine Temperatur des Phosgenstromes von 250 bis 500°C, besonders bevorzugt von 280 bis 330°C eingestellt, wobei der Druck (absolut) dabei typischerweise 500 bis 2.400 mbar, bevorzugt 700 bis 1.500 mbar beträgt.

Im erfindungsgemäßen Verfahren werden die Amine vor Eintritt in den Reaktor bevorzugt auf eine Temperatur des Aminstromes von 200 bis 500°C, besonders bevorzugt 280 bis 350°C eingestellt, wobei der Druck (absolut) dabei typischerweise 500 bis 2.500 mbar, bevorzugt 800 bis 1.600 mbar beträgt.

Im erfindungsgemäßen Verfahren beträgt die mittlere Verweilzeit der Amine im Erhitzer bevorzugt 0,001 bis 60 s, besonders bevorzugt 0,01 bis 10 s.

Im erfindungsgemäßen Verfahren beträgt die mittlere Verweilzeit der Amine im Verdampfer bevorzugt 0,001 bis 60 s, besonders bevorzugt 0,01 bis 10 s.

Im erfindungsgemäßen Verfahren beträgt die mittlere Verweilzeit der Amine im Gasüberhitzer bevorzugt 0,0001 bis 10 s, besonders bevorzugt 0,0005 bis 1 s.

Grundsätzlich kann die jeweilige Erhitzung, Verdampfung und gegebenenfalls Überhitzung unter Einsatz der erfindungsgemäß zu verwendenden Milliwärmetauscher bzw. -verdampfer einstufig oder mehrstufig durch mehrfache Parallel- und/oder Hintereinanderschaltung solcher Milli- und Mikrostrukturbauteile ausgeführt werden. Bei einem mehrstufigen Verfahren kann die Verdampfung auch auf verschiedenen Druck- und Temperaturniveaus durchgeführt werden.

Vorteilhaft an dem erfindungsgemäßen Verfahren ist, dass aufgrund der kurzen Verweilzeiten und daher niedrigen integralen Temperaturbelastungen in den Milli- und Mikrostrukturbauteilen eine Zersetzung der temperaturempfindlichen aliphatischen Amine im Vergleich zu herkömmlichen Verdampfern vermindert oder gar vermieden wird. Außerdem wird bei der Verdampfung durch die geometrisch bedingte Bildung kleiner Blasen das Oberfläche-zu-Volumen-Verhältnis erhöht, weshalb eine sehr effiziente Verdampfung möglich ist. Durch diese Vorteile werden eine höhere Ausbeute und höhere Produktqualität gewährleistet. Weiterhin wird wegen der geringeren Abspaltung von Ammoniak bei der nachfolgenden Phosgenierungsreaktion weniger Ammoniumchlorid gebildet, so dass die Anlage weniger schnell verschmutzt und daher die Laufzeiten zwischen den Reinigungsstillständen verlängert werden können.

Die Eduktströme können nach Verlassen des jeweiligen Verdampfers auch über Einbauten geleitet werden, die eine bessere Vermischung der Reaktanden im Gasraum ermöglichen. Ebensolche Maßnahmen können auch im Reaktor an sich ergriffen werden, um die Vermischung von Amin und Phosgen zu verbessern und somit eine weitgehend störungsfreie kontinuierliche Reaktionsführung zu gewährleisten. Beispiele solcher Maßnahmen sind der Einbau drallerzeugender Einbauten in den Eduktzuleitungen oder ein sich verengender Durchmesser des Reaktorrohres hinter der Zusammenführung von Amin und Phosgenstrom. Weitere geeignete Maßnahmen sind in den im Stand der Technik genannten Anmeldungen zu fmden.

Die Eduktströme können auch mit inerten Verdünnungsmitteln vor der Zuführung in den Reaktionsraum verdünnt werden. Bevorzugtes Inertgas zur Verdünnung ist Stickstoff. Geeignete inerte Lösungsmittel, deren Dämpfe ebenfalls zur Verdünnung des Diamins verwendet werden können, sind beispielsweise Chlorbenzol, o-Dichlorbenzol, Xylol, Chlornaphthalin, Decahydronaphthalin oder deren Gemische.

Die Menge des gegebenenfalls als Verdünnungsmittel mitverwendeten Inertgases oder Lösungsmitteldampfes ist nicht kritisch, kann jedoch genutzt werden um die Verdampfungstemperatur des Amins abzusenken.

Bei der Phosgenierung von Diaminen beträgt der molare Phosgenüberschuss bezogen auf eine Aminogruppe üblicherweise 30 bis 300 %, bevorzugt 60 bis 170 %.

Geeignete zylinderförmige Reaktionsräume sind beispielsweise Rohrreaktoren ohne Einbauten und ohne sich bewegende Teile im Innern des Reaktors. Die Rohrreaktoren bestehen im allgemeinen aus Stahl, Glas, legiertem oder emailliertem Stahl und weisen eine Länge auf, die ausreichend ist, um unter den Verfahrensbedingungen eine vollständige Umsetzung des Amins mit dem Phosgen zu ermöglichen. Die Gasströme werden im allgemeinen an einem Ende des Rohrreaktors in diesen eingeleitet, wobei diese Einleitung beispielsweise durch an einem Ende des Rohrreaktors angebrachte Düsen oder durch eine Kombination aus Düse und einem Ringspalt zwischen Düse und Mischrohr erfolgen kann. Das Mischrohr wird ebenfalls bei einer Temperatur innerhalb des Bereichs von 200 bis 600°C, vorzugsweise 300 bis 500°C, gehalten, wobei diese Temperatur gegebenenfalls durch Beheizen des Reaktionsrohrs aufrechterhalten wird.

Bei der Durchführung des erfindungsgemäßen Verfahrens liegt im allgemeinen der Druck in den Zuleitungen zum Reaktionsraum bei 200 bis 3.000 mbar und am Ausgang aus dem Reaktionsraum bei 150 bis 2.000 mbar, wobei durch Aufrechterhaltung eines geeigneten Differenzdrucks eine Strömungsgeschwindigkeit innerhalb des Reaktionsraums von mindestens 3, vorzugsweise mindestens 6 und besonders bevorzugt 10 bis 120 m/s Sorge getragen wird. Unter diesen Voraussetzungen herrschen innerhalb des Reaktionsraums im allgemeinen turbulente Strömungsverhältnisse vor.

Nach der erfolgten Phosgenierungsreaktion im Reaktionsraum wird das den Reaktionsraum kontinuierlich verlassende, gasförmige Gemisch von dem gebildeten Isocyanat befreit. Dies kann beispielsweise mit Hilfe eines inerten Lösungsmittels erfolgen, dessen Temperatur so gewählt wird, dass sie einerseits oberhalb der Zersetzungstemperatur des dem Isocyanat entsprechenden Carbamidsäurechlorids und andererseits unterhalb der Kondensationstemperatur des Isocyanats und vorzugsweise auch des gegebenenfalls in der Dampfform als Verdünnungsmittel mitverwendeten Lösungsmittels liegt, so dass Isocyanat und Hilfslösungsmittel kondensieren bzw. sich in dem Lösungsmittel lösen, während überschüssiges Phosgen, Chlorwasserstoff und gegebenenfalls als Verdünnungsmittel mitverwendetes Inertgas die Kondensationsstufe bzw. das Lösungsmittel gasförmig durchlaufen. Zur selektiven Gewinnung des Isocyanats aus dem den Reaktionsraum gasförmig verlassenden Gemisch besonders gut geeignet sind bei einer Temperatur von 120 bis 200°C, vorzugsweise 120 bis 170°C, gehaltene Lösungsmittel der oben beispielhaft genannten Art, insbesondere technisches Dichlorbenzol. Denkbare Methoden der selektiven Kondensation des gebildeten Isocyanats aus dem den Reaktor verlassenden Gasgemisch unter Verwendung derartiger Lösungsmittel sind beispielsweise das Durchleiten des Gasgemisches durch das genannte Lösungsmittel oder das Eindüsen des Lösungsmittels (Lösungsmittelnebel) in den Gasstrom.

Das die Kondensationsstufe zur Gewinnung des Isocyanats durchlaufende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Lösungsmittel (z.B. Chlorbenzol oder Dichlorbenzol) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recyclisiert werden.

Die Reindarstellung der Isocyanate erfolgt am Besten durch destillative Aufarbeitung der Lösung des Isocyanats in dem zur Isocyanatkondensation eingesetzten Lösungsmittel.

**Beispiele:**

Die prinzipielle Eignung der Milliwärmetauscher zur schonenden Verdampfung und Überhitzung von Aminen wurde in einer Versuchsanlage nachgewiesen. Als Amine wurden 1,6-Diaminohexan (HDA), 1-Amino-3,5,5-thmethyl-5-aminomethylcyclohexan (IPDA) und 4,4'-Diaminodicyclohexylmethan (PACM 20) verwendet.

Zur Erhitzung, Verdampfung und Überhitzung wurden jeweils mehrere hintereinander angeordnete Milliwärmetauscher mit jeweils rechteckigen Kanälen zur Strömungsführung eingesetzt. Die Kanäle zur Strömungsführung hatten eine Innenhöhe von 3,1 mm, eine Innenbreite von 18 mm und waren mit einer Schichtstruktur gefüllt. Diese Füllung bestand aus drei Schichten mit einer Höhe von jeweils 1 mm. Die Gesamtlänge der Kanäle je Verdampfer betrug 300 mmm. Die Wärmeübertragungsfläche (arithmetisches Mittel zwischen Innen- und Außenwandfläche) pro Kanal betrug 156 cm$^2$ und das freie Innenvolumen lag bei 12,8 cm$^3$.

Für die Erhitzung wurden drei solcher Milliwärmetauscher zu einem Gegenstromwärmetauscher in Serie geschaltet (MWT 1 - MWT 3).

Für die Verdampfung wurden zwei dieser Milliwärmetauscher zu einem Gegenstromwärmetauscher in Serie geschaltet (MWT 4 - MWT 5).

Alle Milliwärmetauscherapparate hatten einen inneren Manteldurchmesser von ca. 40 mm und waren im vom Wärmeträgeröl durchströmten Mantelraum mit mehreren Umlenkblechen bestückt.

Bei der Erhitzung wurden die Amine in der ersten Wärmetauscherserie (MWT 1 - MWT 3) von 60°C bis zum Siedepunkt erhitzt und dann in der zweiten Wärmetauscherserie (MWT 4 - MWT 5) verdampft und überhitzt. Im nachgeschalteten Kühler wurde das Amin kondensiert, in die Vorlage geleitet und anschließend erneut in den Kreislauf gepumpt.

Um chemische Änderungen der Amine zu verfolgen, wurden in regelmäßigen Abständen Proben mittels Gaschromatographie und Ammoniak-Analytik untersucht.

Ein bei konventionellen Wärmetauschern aufgrund von Ablagerungen im Laufe der Zeit entstehender Druckaufbau wurde während der Versuchsdauer bei keinem der eingesetzten Amine beobachtet.

**Beispiel 1**

HDA wurde in den auf 224°C aufgeheizten MWT 1 - MWT 3 auf 217°C bei einem Druck von 2,3 bara (Druck in bar absolut) erhitzt und in den auf 307°C aufgeheizten MWT 4 - MWT 5 verdampft und auf 305°C bei einem Druck von 1,0 bara überhitzt. Bei einer Umpumpmenge von 20 kg/h betrug die mittlere Verweilzeit im MWT 1 - MWT 3 4,7 s und im MWT 4 - MWT 5 9,4 s unter der Annahme einer vollständigen Flüssigkeitsströmung bis zum Austritt. Die reale Verweilzeit lag durch die Verdampfung noch deutlich unter diesem Wert. Nach 80 statistischen Durchgängen nahm die Konzentration an Nebenkomponenten von 170 ppm auf 270 ppm zu.

Als Wärmedurchgangskoeffizienten wurden bestimmt: 1.200 bis 1.700 W/(m$^2$K) für die Erhitzung zum Siedepunkt bei Umpumpmengen von 20 bis 40 kg/h, 1.800 W/(m$^2$K) für die Verdampfung bei einer Umpumpmenge von 40 kg/h und 100 bis 500 W/(m$^2$K) für die Überhitzung bei Umpumpmengen von 5 bis 20 kg/h.

**Beispiel 2**

IPDA wurde in den auf 277°C aufgeheizten MWT 1 - MWT 3 auf 260°C bei einem Druck von 1,6 bara erhitzt und in den auf 305°C aufgeheizten MWT 4 - MWT 5 verdampft und auf 302°C bei einem Druck von 1,0 bara überhitzt. Bei einer Umpumpmenge von 20 kg/h betrug die mittlere Verweilzeit im MWT 1 - MWT 3 5,2 s und im MWT 4 - MWT 5 10,5 s unter der Annahme einer vollständigen Flüssigkeitsströmung bis zum Austritt. Die reale Verweilzeit lag durch die Verdampfung noch deutlich unter diesem Wert. Nach 80 statistischen Durchgängen nahm die Konzentration an Nebenkomponenten von 1.300 ppm auf 2.200 ppm zu.

Als Wärmedurchgangskoeffizienten wurden bestimmt: 500 bis 1.650 W/(m$^2$K) für die Erhitzung zum Siedepunkt bei Umpumpmengen von 10 bis 110 kg/h, 1.800 W/(m$^2$K) für die Verdampfung bei einer Umpumpmenge von 20 kg/h und 200 bis 300 W/(m$^2$K) für die Überhitzung bei Umpumpmengen von 10 bis 15 kg/h.

**Beispiel 3**

PACM 20 wurde in den auf 338°C aufgeheizten MWT 1 - MWT 3 auf 327°C bei einem Druck von 1,2 bara erhitzt und in den auf 352°C aufgeheizten MWT 4 - MWT 5 verdampft und auf 335°C bei einem Druck von 1,0 bara überhitzt. Bei einer Umpumpmenge von 15 kg/h betrug die mittlere Verweilzeit im MWT 1 - MWT 3 7 s und im MWT 4 - MWT 5 14 s, unter der Annahme einer vollständigen Flüssigkeitsströmung bis zum Austritt. Die reale Verweilzeit lag durch die Verdampfung noch deutlich unter diesem Wert. Nach 60 statistischen Durchgängen nahm die Konzentration an Nebenkomponenten von 3.900 ppm auf 4.400 ppm zu.

Als Wärmedurchgangskoeffizienten wurden bestimmt: 350 bis 1.850 W/(m²K) für die Erhitzung zum Siedepunkt bei Umpumpmengen von 10 bis 100 kg/h, 900 W/(m²K) für die Verdampfung bei einer Umpumpmenge von 15 kg/h und 250 W/(m²K) für die Überhitzung bei einer Umpumpmenge von 15 kg/h.

**Patentansprüche**

1. Verfahren zur Herstellung von Isocyanaten durch Phosgenierung von Aminen in der Gasphase, bei dem zur Flüssigerhitzung, Verdampfung und/oder Gasüberhitzung der Amine ein oder mehrere Wärmetauscher mit einer volumenspezifischen Wärmetauscherfläche fiir die Aminseite von mindestens 1.000 m²/m³ eingesetzt werden, die zur Strömungsführung der Amine Kanäle aufweisen, die einen hydraulischen Durchmesser von 1000 bis 10.000 μm besitzen.

2. Verfahren gemäß Anspruch 1, **dadurch gekennzeichnet, dass** als Wärmetauscher Milliwärmetauscher vom Typ Stapelkanalmikrowärmetauscher oder Millikanalrohrwärmetauscher eingesetzt werden mit einem hydraulischen Durchmesser der Kanäle zur Führung des Aminstromes von 2.000 bis 5.000 μm und einer Länge der Kanalrohr von jeweils 10 bis 400 cm.

3. Verfahren gemäß Anspruch 1 oder 2 **dadurch gekennzeichnet, dass** die auf das Volumen der Aminkanäle bezogene Wärmeaustauschfläche 1* 10³ bis 1 * 10⁵ m²/m³ beträgt.

4. Verfahren gemäß einem der Ansprüche 1 bis 3, **dadurch gekennzeichnet, dass** die Kanäle zur Strömungsführung und/oder die Kanäle bzw. der Raum zur Führung des Heizmediums Einbauten enthalten.

5. Verfahren gemäß einem der Ansprüche 1 bis 4, **dadurch gekennzeichnet, dass** die mittlere Verweilzeit der Amine im Erhitzer und/oder Verdampfer jeweils 0,01 bis 10 s und /oder im Gasüberhitzer 0,0005 bis 1 s beträgt.

6. Verfahren gemäß einem der Ansprüche 1 bis 5, **dadurch gekennzeichnet, dass** die Amine vor Eintritt in den Reaktor auf eine Temperatur des Aminstromes von 280 bis 350°C bei einem Druck (absolut) von 800 bis 1.600 mbar erhitzt werden.

7. Verfahren gemäß einem der Ansprüche 1 bis 6, **dadurch gekennzeichnet, dass** das Phosgen vor Eintritt in den Reaktor auf eine Temperatur des Phosgenstromes von 280 bis 330°C bei einem Druck (absolut) von 700 bis 1.500 mbar erhitzt werden.

8. Verfahren gemäß einem der Ansprüche 1 bis 7, **dadurch gekennzeichnet, dass** der molare Phosgenüberschuss bezogen pro zu phosgenierende Aminogruppe 60 bis 170 % beträgt.

9. Verfahren gemäß einem der Ansprüche 1 bis 8, **dadurch gekennzeichnet, dass** als Amine Isophorondiamin (IPDA), Hexamethylendiamin (HDA), Bis(p-aminocyclohexyl)methan, (PACM 20) oder 1,8-Diamino-4-(aminomethyl)octan (Triaminononan) eingesetzt werden.

**Claims**

1. Process for preparing isocyanates by phosgenation of amines in the gas phase, in which one or more heat exchangers which have a heat transfer area per unit volume for the amine side of at least 1000 m²/m³ and have channels having a hydraulic diameter of from 1000 to 10 000 μm for the flow of the amines are used for liquid heating, vaporization and/or gas superheating of the amines.

2. Process according to Claim 1, **characterized in that** milli heat exchangers of the stacked channel micro heat exchanger or milli channel tube heat exchanger type having a hydraulic diameter of the channels for the flow of the amine stream of from 2000 to 5000 μm and a length of the channel tubes of from 10 to 400 cm each are used as heat exchangers.

3. Process according to Claim 1 or 2, **characterized in that** the heat transfer area per unit volume of the amine channels is from 1 * 10³ to 1 * 10⁵ m²/m³.

4. Process according to any of Claims 1 to 3, **characterized in that** the flow channels and/or the channels or the space for conveying the heating medium contain internals.

5. Process according to any of Claims 1 to 4, **characterized in that** the mean residence time of the amines in the heater and/or vaporizer is in each case from 0.01 to 10 s and/or that in the gas superheater is from 0.0005 to 1 s.

6. Process according to any of Claims 1 to 5, **characterized in that** the amines are heated to a temperature of the amine stream of from 280 to 350°C at an (absolute) pressure of from 800 to 1600 mbar before entering the reactor.

7. Process according to any of Claims 1 to 6, **characterized in that** the phosgene is heated to a temperature of the phosgene stream of from 280 to 330°C at an (absolute) pressure of from 700 to 1500 mbar before entering the reactor.

8. Process according to any of Claims 1 to 7, **characterized in that** the molar excess of phosgene per amino group to be phosgenated is from 60 to 170%.

9. Process according to any of Claims 1 to 8, **characterized in that** isophoronediamine (IPDA), hexamethylenediamine (HDA), bis(p-aminocyclohexyl) methane (PACM 20) or 1,8-diamino-4-(aminomethyl)octane (triaminononane) are used as amines.

# Revendications

1. Procédé pour la préparation d'isocyanates par phosgénation d'amines en phase gazeuse, dans lequel on utilise, pour le chauffage des liquides, l'évaporation et/ou la surchauffe des gaz des amines, un ou plusieurs échangeurs de chaleur avec une surface d'échange thermique spécifique au volume pour le côté amines d'au moins 1 000 m$^2$/m$^3$, lesquels présentent, pour l'introduction de l'écoulement des amines, des canaux qui possèdent un diamètre hydraulique de 1 000 à 10 000 $\mu$m.

2. Procédé selon la revendication 1, **caractérisé en ce que** l'on utilise comme échangeur de chaleur, des milli-échangeurs de chaleur de type micro-échangeur de chaleur à canaux empilés ou échangeurs de chaleur tubulaires à milli-canaux avec un diamètre hydraulique des canaux pour l'introduction du courant des amines de 2 000 à 5 000 $\mu$m et une longueur des tubes des canaux à chaque fois de 10 à 400 cm.

3. Procédé selon la revendication 1 ou 2, **caractérisé en ce que** la surface d'échange thermique rapportée au volume des canaux pour les amines est de 1 x 10$^3$ à 1 x 10$^5$ m$^2$/m$^3$.

4. Procédé selon l'une quelconque des revendications 1 à 3, **caractérisé en ce que** les canaux pour l'introduction de l'écoulement et/ou les canaux respectivement l'espace pour l'introduction du milieu chauffant contiennent des chicanes.

5. Procédé selon l'une quelconque des revendications 1 à 4, **caractérisé en ce que** le temps de séjour moyen des amines dans le dispositif de chauffage et/ou l'évaporateur est à chaque fois de 0,01 à 10 s et/ou dans le dispositif de surchauffe des gaz de 0,0005 à 1 s.

6. Procédé selon l'une quelconque des revendications 1 à 5, **caractérisé en ce que** les amines sont, avant l'entrée dans le réacteur, chauffées à une température du courant des amines de 280 à 350°C à une pression (absolue) de 800 à 1 600 mbar.

7. Procédé selon l'une quelconque des revendications 1 à 6, **caractérisé en ce que** le phosgène est chauffé, avant l'entrée dans le réacteur, à une température du courant du phosgène de 280 à 330°C à une pression (absolue) de 700 à 1 500 mbar.

8. Procédé selon l'une quelconque des revendications 1 à 7, **caractérisé en ce que** l'excès molaire en phosgène rapporté au groupe amino à phosgéner est de 60 à 170 %.

9. Procédé selon l'une quelconque des revendications 1 à 8, **caractérisé en ce que** l'on utilise comme amines l'isophoronediamine (IPDA), l'hexaméthylènediamine (HDA), le bis(p-aminocyclohexylméthane), (PACM 20) ou le 1,8-diamino-4-(aminométhyl)octane (triaminononane).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0289840 A
- EP 928785 A
- EP 1319655 A
- EP 1555258 A
- EP 1275639 A
- EP 1275640 A
- EP 1403248 A
- EP 1526129 A
- WO 2005016512 A
- EP 1284159 A
- EP 486749 A1

**In der Beschreibung aufgeführte Nicht-Patentliteratur**

- **K. SCHUBERT ; J. BRANDNER ; M. FICHTNER ; G. LINDER ; U. SCHYGULLA ; A. WENKA.** Microstructure devices for applications in thermal and chemical process engineering, Heat and Transport Phenomena in Microsystems. *Proc. Of the Internat. Conf.,* 15. Oktober 2000